## Europäisches Patentamt

## European Patent Office

## Office européen des brevets

(19)

(11) **EP 1 351 716 B1**

(12) **EUROPÄISCHE PATENTSCHRIFT**

(45) Veröffentlichungstag und Bekanntmachung des Hinweises auf die Patenterteilung:
**29.06.2005 Patentblatt 2005/26**

(21) Anmeldenummer: **02715389.9**

(22) Anmeldetag: **04.01.2002**

(51) Int Cl.⁷: **A61L 9/01**, A61L 9/04

(86) Internationale Anmeldenummer:
**PCT/EP2002/000040**

(87) Internationale Veröffentlichungsnummer:
**WO 2002/055114 (18.07.2002 Gazette 2002/29)**

(54) **VERFAHREN ZUR UNTOXISCHEN GERUCHSNEUTRALISIERUNG VON LUFT**

METHOD FOR NEUTRALISING ODOURS IN THE AIR IN A NON-TOXIC MANNER

PROCEDE POUR NEUTRALISER DES ODEURS DANS L'AIR DE MANIERE NON TOXIQUE

(84) Benannte Vertragsstaaten:
**AT BE CH CY DE DK ES FI FR GB GR IE IT LI LU MC NL PT SE TR**

(30) Priorität: **09.01.2001 DE 10100595**

(43) Veröffentlichungstag der Anmeldung:
**15.10.2003 Patentblatt 2003/42**

(73) Patentinhaber: **Schür, Jörg-Peter, Prof.**
**41844 Wegberg-Dalheim (DE)**

(72) Erfinder: **Schür, Jörg-Peter, Prof.**
**41844 Wegberg-Dalheim (DE)**

(74) Vertreter: **Helbing, Jörg, Dr. Dipl.-Chem. et al**
**Patentanwälte**
**von Kreisler-Selting-Werner,**
**Postfach 10 22 41**
**50462 Köln (DE)**

(56) Entgegenhaltungen:
**EP-A- 0 101 083          GB-A- 1 571 517**
**US-A- 4 808 396**

- **DATABASE WPI Derwent Publications Ltd., London, GB; AN 78-33903A XP002193606 & JP 53 032134 A (KATSURAYA FINE GOODS), 27. März 1978 (1978-03-27)**
- **DATABASE WPI Derwent Publications Ltd., London, GB; AN 86-136554 XP002193607 & SU 1 189 454 A (URAL VNIPI KHIM PROMY), 7. November 1985 (1985-11-07)**
- **PATENT ABSTRACTS OF JAPAN vol. 014, no. 453 (C-0764), 28. September 1990 (1990-09-28) & JP 02 180267 A (MATSUSHITA ELECTRIC WORKS LTD), 13. Juli 1990 (1990-07-13)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur untoxischen Geruchsneutralisierung, umfassend das Verteilen oder Zerstäuben einer speziellen geruchsmaskierenden Zusammensetzung, für diesen Zweck geeignete geruchsmaskierende Zusammensetzungen sowie die Verwendung dieser Zusammensetzungen zur Geruchsreduzierung.

Unangenehme Gerüche sind ein grundsätzliches Problem der heutigen hochtechnisierten Zivilisation, insbesondere dann, wenn keine ausreichende Durchlüftung der Raumumgebung möglich ist. Darüber hinaus stehen unangenehme Gerüche in der Raumluft unmittelbar mit einer gleichzeitigen Verkeimung in Zusammenhang. Zwar wurde die Verkeimung der Raumluft, die ein grundsätzliches Problem sowohl in privaten Haushalten und in gewerblichen Bürokomplexen als auch in Betrieben des produzierenden Gewerbes, insbesondere in lebensmittelverarbeitenden Betrieben ist, in den Anmeldungen DE 19931185.4 und PCT/EP00/06462 durch Verteilen und Verstäuben einer antimikrobiellen Zusammensetzung, die einen oder mehrere GRAS(Generally Recognized As Safe)-Aromastoffe oder deren Derivate enthält, gelöst. Geeignete Vorrichtungen für dieses Verteilen und Verstäuben sind z. B. in der PCT/EP00/02992 beschrieben. Das Problem der Geruchsbelastung, insbesondere dann, wenn diese einmal aufgetreten sind, wurde hierin jedoch nicht gelöst. Sie ist zum gegenwärtigen Zeitpunkt, falls überhaupt, einzig und allein durch raschen Luftaustausch zu entgegnen. Der hierdurch erzielte Effekt ist jedoch nur unzureichend.

GB-A-1 571 517 betrifft ein Verfahren zur Geruchsneutralisierung, welches Polyvinylpyrrolidon verwendet. SU 1 189 454 betrifft eine lufterfrischende Zusammensetzung, die Polyvinylpyrrolidon und Duftstoff, Citral und Geraniol enthält, um die duftmaskierende Wirkung zu erhöhen. Eine ursächliche Geruchsbelästigung durch Mikroorganismen sowie eine Entfernung sowohl der Geruchsbelästigung als auch der Mikroorganismen wird in keiner dieser drei Dokumente beschrieben. JP-A-53032134 betrifft eine temperaturstabilisierte Duftgelzusammensetzung, die Polyvinylpyrrolidon nicht als geruchsmaskierende Komponente, sondern nur als viskositätserhöhendes Agens enthält. DE-C-4322750 offenbart ein konditionierendes Haarbehandlungsmittel, das neben einem Vinylpyrrolidon-Polymer zwingend 0,1 bis 10 Gew.-% einer Naphthalinsulfonsäure und/oder deren Salz enthält.

Überraschenderweise wurde nun gefunden, dass durch Verteilen/Zerstäuben einer speziellen geruchsmaskierenden Zusammensetzung die Geruchsbelastung in der Raumluft signifikant verringert werden kann. Darüber hinaus kann durch den Einsatz von Functional-Flavour- und Duftstoffkomponenten die subjektive Belastung der Raumluft bzw. deren bakterielle Belastung verringert werden. Gegenstand der vorliegenden Anmeldung ist demgemäß

(1) ein Verfahren zur untoxischen Geruchsreduzierung, umfassend das Verteilen oder Zerstäuben einer geruchsmaskierenden Zusammensetzung in der zu behandelnden Umgebung, wobei die geruchsmaskierende Zusammensetzung wenigstens eine geruchsmaskierende Komponente (A) ausgewählt aus Maisstärke, Mangansalzen und Polyvinylpyrrolidon und eine Aromastoffkomponente (C), ausgewählt aus ätherischen Ölen, Aromastoffen und Duftstoffen, enthält, wobei jedoch die geruchsmaskierende Komponente (A) wenigstens Polyvinylpyrrolidon und die Aromastoffkomponente (C) wenigstens den GRAS(Generally Recognized As Safe in Food)-Aromastoff (gemäß der FEMA/FDA GRAS-Flavour-Substances-Liste 3 - 15 Nr. 2001 - 3905 (Stand 2000)) Benzylalkohol enthält;

(2) eine bevorzugte Ausführungsform des in (1) definierten Verfahrens, wobei die geruchsmaskierende Zusammensetzung weiterhin eine Functional-Flavour-Komponente (B) enthält;

(3) eine geruchsmaskierende Zusammensetzung, insbesondere eine solche Zusammensetzung zur untoxischen Geruchsreduzierung, wie in (1) bis (2) definiert, vorausgesetzt dass die Zusammensetzung, wenn sie 0,5 - 5,0 Gew.-% Polyvinylpyrrolidon enthält, nicht 0,1 - 10,0 Gew.-% einer Naphthalinsulfonsäure und/oder deren Salze enthält; und

(4) die Verwendung der in (3) definierten Zusammensetzung zur untoxischen Geruchsreduzierung.

Figuren

Die nachfolgend erwähnten Figuren zeigen Vorrichtungen, die in den erfindungsgemäßen Geruchsreduzierungsverfahren einsetzbar sind.

Fig. 1 zeigt einen Luft-EvL(Entkeimung von Luft)-Bubbler.
Fig. 2 zeigt ein Zweistoffdüsensystem.
Fig. 3 zeigt ein EvL-Verdampfungssystem.
Fig. 4 zeig eine Bubbler-EvL-Vorrichtung für die Entkeimung in der Verpackung.
Fig. 5 zeigt eine schematische Seitenansicht der Vorrichtung zur Anreicherung von Luft.
Fig. 6 zeigt eine der in Fig. 5 dargestellten Vorrichtung entsprechende Vorrichtung mit nachgeschalteter Druckerzeugungseinrichtung.
Fig. 7 zeigt ein EvL-Docht-System mit Heizplatine und Ventilator.

Im folgenden werden die Bestandteile der erfindungsgemäßen Zusammensetzungen näher beschrieben:

Die bevorzugte Verbindung der geruchsmaskierenden Komponente (A) ist Polyvinylpyrrolidon (Polyvidone;  Poly(2-oxo-1-Pyrrolidinyl)ethylen;  Poly(1-vinyl-2-Pyrrolidon; nachfolgend auch kurz "PVP"), insbesondere solches PVP mit einer Molmasse von 10.000 bis 60.000 g/mol, vorzugsweise 30.000 bis 50.000 g/mol. Besonders bevorzugt ist PVP mit einer Molmasse von etwa 40.000 g/mol, d.h. es ist ein PVP, das einen gewissen Vernetzungsgrad (d. h. eine Viskosität von 15 bis 25, vorzugsweise etwa 2 mPa•s (20 Gew.-% in Wasser) besitzt. Der Anteil der geruchsmaskierenden Komponente (A) an der geruchsmaskierenden Zusammensetzung liegt vorzugsweise im Bereich von 0,001 bis 50 Gew.-%, besonders bevorzugt 0,1 bis 10 Gew.-%.

Gemäß der Ausführungsform (2) enthält die geruchsmaskierende Zusammensetzung eine weitere Functional-Flavour-Komponente (B), und diese enthält vorzugsweise eine oder mehrere der folgenden Substanzen:

Hexylbutyrat, Octylacetat, Isobutylisobutyrat, cis-3-Hexen-1-ylacetat cis-3, γ-Decalactone, Ethylcaproat, Butylacetat, Ethylbenzoat, Ethylbutyrat, Hexylacetat, Methylcaproat, Phenylethylalkohol, Citronellol, Undecylaldehyd, Benzylphenylacetat, Cinnamik-Alkohol, Eugenol, Benzylacetat, Linalool, cis-Jasmone, Acetylmethylanthranilat, cis-3-Hexen-1-ol, cis-3-Hexen-1-ylsalicylat, Methylbenzoat, Methylsalicylat, Geranylacetat, cis-3-Hexen-1-ylacetat, Litsea Cubeba, Phenylpropylalkohol und Phenylethylacetat.

Vorzugsweise beträgt der Anteil der Functional-Flavour-Komponente (B) 0,001 bis 20 Gew.-%, vorzugsweise 0,1 bis 5 Gew.-%, der geruchsmaskierenden Zusammensetzung.

Gemäß der Ausführungsform (1) der Erfindung enthält die geruchsmaskierende Zusammensetzung noch weiterhin eine Aromastoffkomponente (C), die ausgewählt ist aus ätherischen Ölen, Aromastoffen und Duftstoffen und wenigstens Benzylalkohol enthält. Dabei ist der Anteil der Aromastoffkomponente (C) an der geruchsmaskierenden Zusammensetzung 0,01 bis 95 Gew.-%, vorzugsweise 0,1 bis 80 Gew.-%. In einer besonders bevorzugten Ausführungsform enthält die Aromastoffkomponente (C) antimikrobielle Substanzen, vorzugsweise enthält sie wenigstens einen GRAS (Generally Recognized As Safe)-Aromastoff. Besonders bevorzugt hiervon sind solche Aromastoffkomponenten (C), die einen aromatischen GRAS-Aromaalkohol (wie Benzylalkohol, Zimtalkohol, α-Methylbenzylalkohol und Anisalkohol, wobei Benzylalkohol bevorzugt ist) oder eine GRAS-Polyphenolverbindung enthalten, bzw. solche, die wenigstens 2 GRAS-Aromastoffe enthalten. Es hat sich dabei gezeigt, dass besonders solche Aromastoffkomponenten (C), die

(a) eine oder mehrere GRAS-Aroma-Alkohole oder deren Derivate und
(b) einen oder mehrere Aromastoffe, ausgewählt aus

(b1) Polyphenolverbindungen und
(b2) GRAS-Aromasäuren oder deren Derivate enthalten,

besonders bevorzugt sind.

Die genannten GRAS-Aroma-Alkohole der Komponente (a) sowie die nachfolgend definierten Komponenten (b) bis (h) sind von der FDA-Behörde zur Verwendung in Nahrungsmitteln als gewerbesicher anerkannt (GRAS = Generally Recognized As Safe In Food). Bei den erwähnten GRAS-Aroma-Alkoholen und auch bei den nachfolgend definierten anderen GRAS-Aromastoffen handelt es sich um solche Verbindungen, die in FEMA/FDA GRAS Flavour Substances Lists GRAS 3-15 Nr. 2001-3905 (Stand 2000) genannt sind. In dieser Liste sind natürliche und naturidentische Aromastoffe aufgeführt, die von der amerikanischen Gesundheitsbehörde FDA zur Verwendung in Nahrungsmitteln zugelassen sind: FDA Regulation 21 CFR 172.515 für naturidentische Aromastoffe (Synthetic Flavoring Substances and Adjuvants) und FDA Regulation 21 CFR 182.20 für natürliche Aromastoffe (Natural Flavoring Substances and Adjuvants).

Die Aromastoffkomponente (C) kann

0,1 bis 99,9 Gew.-%, vorzugsweise 0,5 bis 99 Gew.-%, Komponente (a),
0 bis 25 Gew.-%, vorzugsweise 0,01 bis 10 Gew.-%, Komponente (b1) und/oder
0 bis 70 Gew.-%, vorzugsweise 0,01 bis 30 Gew.-%, Komponente (b2)

enthalten.

Erfindungsgemäß kann die Komponente (a) einen oder mehrere GRAS-Aroma-Alkohole enthalten. Bevorzugt wird erfindungsgemäß der Einsatz von zwei oder drei GRAS-Aroma-Alkoholen. Im einzelnen können beispielsweise folgende GRAS-Aroma-Alkohole zum Einsatz kommen:

Benzylalkohol, Acetoin (Acetylmethylcarbinol), Ethylalkohol (Ethanol), Propylalkohol (1-Propanol), iso-Propylalkohol (2-Propanol, Isopropanol), Propylenglykol, Glycerin, n-Butylalkohol (n-Propylcarbinol), iso-Butylalkohol (2-Methyl-1-propanol), Hexylalkohol (Hexanol), L-Menthol, Octylalkohol (n-Octanol), Zimtalkohol (3-Phenyl-2-propen-1-ol), α-Methylbenzylalkohol (1-Phenylethanol), Heptylalkohol (Heptanol), n-Amylalkohol (1-Pentanol), iso-Amylalkohol (3-Methyl-1-butanol), Anisalkohol (4-Methoxybenylalkohol, p-Anisalkohol), Citronellol, n-Decylalkohol (n-Decanol), Geraniol, β-γ-Hexanol (3-Hexenol), Laurylalkohol (Dodecanol), Linalool, Nerolidol, Nonadienol (2,6-Nonadien-1-ol), Nonylalkohol (Nonanol-1), Rhodinol, Terpineol, Borneol, Clineol (Eucalyptol), Anisol, Cuminylalkohol (Cuminol), 10-Undecen-1-ol, 1-Hexadecanol. Als Derivate können sowohl natürliche oder naturidentische Derivate als auch synthetische Derivate eingesetzt werden. Geeignete Derivate sind z. B. die Ester, Ether und Carbonate der vorstehend genannten GRAS-Aroma-Alkohole. Besonders bevorzugte GRAS-Aroma-Alkohole sind Benzylalkohol, 1-Propanol, Glycerin, Propylenglycol, n-Butylalkohol, Citronellol, Hexanol, Linalool, Acetoin und deren Derivate.

Als Komponente (b1) können die folgenden Polyphenole eingesetzt werden:

Brenzcatechin, Resorcin, Hydrochinon, Phloroglucin, Pyrogallol, Cyclohexan, Usninsäure, Acylpolyphenole, Lignine, Anthocyane, Flavone, Catechine, Gallussäurederivate (z. B. Tannine, Gallotannin, Gerbsäuren, Gallus-Gerbsäuren), (einschließlich der Derivate der vorstehend genannten Verbindungen wie (2,5-Dihydroxyphenyl)carboxyl- und (2,5-Dihydroxyphenyl)alkylencarboxylsubstitutionen, Salze, Ester, Amide), Kaffesäure und deren Ester und Amide, Flavonoide (z. B. Flavon, Flavonol, Isoflavon, Gossypetin, Myrecetin, Robinetin, Apigenin, Morin, Taxifolin, Eriodictyol, Naringin, Rutin, Hesperidin, Troxerutin, Chrysin, Tangeritin, Luteolin, Catechine, Quercetin, Fisetin, Kaempferol, Galangin, Rotenoide, Aurone, Flavonole, -diole), Extrakte aus z. B. Camellia Primula. Weiterhin können auch deren mögliche Derivate, z. B. Salze, Säuren, Ester, Oxide und Ether verwendet werden. Das besonders bevorzugte Polyphenol ist Tannin (eine GRAS-Verbindung).

Als Komponente (b2) können beispielsweise folgende GRAS-Säuren zum Einsatz kommen:

Essigsäure, Aconitsäure, Adipinsäure, Ameisensäure, Apfelsäure (1-Hydroxybernsteinsäure), Capronsäure, Hydrozimtsäure (3-Phenyl-1-propionsäure), Pelargonsäure (Nonansäure), Milchsäure (2-Hydroxypropionsäure), Phenoxyessigsäure (Glykolsäurephenylether), Phenylessigsäure ($\alpha$-Toluolsäure), Valeriansäure (Pentansäure), iso-Valeriansäure (3-Methylbutansäure), Zimtsäure (3-Phenylpropensäure), Citronensäure, Mandelsäure (Hydroxyphenylessigsäure), Weinsäure (2,3-Dihydroxybutandisäure; 2,3-Dihydroxybernsteinsäure), Fumarsäure, Tanninsäure und deren Derivate.

Geeignete Derivate der genannten Säuren im Sinne der vorliegenden Erfindung sind Ester (z. B. $C_{1-6}$-Alkylester und Benzylester), Amide (einschließlich N-substituierte Amide) und Salze (Alkali-, Erdalkali- und Ammoniumsalze). Ebenfalls umfaßt der Begriff Derivate im Sinne der vorliegenden Erfindung Modifikationen der Seitenketten-Hydroxyfunktionen (z. B. Acyl- und Alkylderivate) und Modifikationen der Doppelbindungen (z. B. die perhydrierten und hydroxilierten Derivate der genannten Säuren).

Das Mischungsverhältnis der Komponente (a) zu Komponenten (b) liegt vorzugsweise zwischen 10.000 : 1 und 1 : 10.000, besonders bevorzugt zwischen 1000 : 1 und 1:1000 und ganz besonders bevorzugt zwischen 100 : 1 und 1 : 100.

Eine bevorzugte Ausführungsform des erfindungsgemäßen Verfahrens ist die Aromastoffkomponente (C') die

(a1) Benzylalkohol als notwendigen Bestandteil und gegebenenfalls
(a2) einen oder mehrere weitere GRAS-Aroma-Alkohole oder deren Derivate und
(b1) eine oder mehrere Polyphenolverbindungen und/oder
(b2) eine oder mehrere GRAS-Säuren oder deren Derivate

enthält.

Geeignete Mengen der Komponenten (a1), (a2), (b1) und (b2) sind dabei:

0,1 bis 99 Gew.-%, vorzugsweise 0,1 bis 75 Gew.-% Benzylakohol;
0 bis 99,8 Gew.-%, vorzugsweise 0,01 bis 99 Gew.-% Komponente (a2);
0 bis 25 Gew.-%, vorzugsweise 0,01 bis 10 Gew.-% Komponente (b1) und/oder
0 bis 70 Gew.-%, vorzugsweise 0,01 bis 30 Gew.-% Komponente (b2).

Die Aromastoffkomponente (C') kann weiterhin noch die folgenden Komponenten (c) bis (h) enthalten, die ebenfalls Aromastoffe sind, die in der FEMA/FDA GRAS Flavour Substances Liste als G.R.A.S. (Generally Recognized As Safe In Food) 3-15 Nr. 2001-3905 (Stand 2000) anerkannt sind.

Als Komponente (c) können folgende Phenolverbindungen zum Einsatz kommen: Thymol, Methyleugenol, Acetyleugenol, Safrol, Eugenol, Isoeugenol, Anethol, Phenol, Methylchavicol (Estragol; 3-4-Methoxyphenyl-1-propen), Carvacrol, $\alpha$-Bisabolol, Fomesol, Anisol (Methoxybenzol) und Propenylguaethol (5-Prophenyl-2-ethoxaphenol) und deren Derivate.

Als GRAS-Ester (Komponente (d)) kommen Allicin und die folgenden Acetate iso-Amylacetat (3-Methyl-1-butylacetat), Benzylacetat, Benzylphenylacetat, n-Butylacetat, Cinnamylacetat (3-Phenylpropenylacetat), Citronellylacetat, Ethylacetat (Essigester), Eugenolacetat (Acetyleugenol), Geranylacetat, Hexylacetat (Hexanylethanoat), Hydrocinnamylacetat (3-Phenylpropylacetet), Linalylacetat, Octylacetat, Phenylethylacetat, Terpinylacetat, Triacetin (Glyceryltriacetat), Kaliumacetat, Natriumacetat, Calciumacetat zum Einsatz. Weitere geeignete Ester sind die Esterderivate der vorstehend definierten Säuren (Komponente (b2)).

Als Terpene (Komponente (e)) kommen z. B. Campher, Limonen und β-Caryophyllen in Betracht.

Zu den verwendbaren Acetalen (Komponente (f)) zählen z. B. Acetal, Acetaldehyddibutylacetal, Acetaldehyddipropylacetal, Acetaldehydphenethylpropylacetal, Zimtaldehydethylenglycolacetal, Decanaldimethylacetal, Heptanaldimethylacetal, Heptanalglycerylacetal und Benzaldehydpropylenglykolacetal.

Als Aldehyde (Komponente (g)) sind z. B. Acetylaldehyd, Anisaldehyd, Benzaldehyd, iso-Butylaldehyd (Methyl-1-propanal), Citral, Citronellal, n-Caprinaldehyd (n-Decanal), Ethylvanillin, Fufurol, Heliotropin (Piperonal), Heptylaldehyd (Heptanal), Hexylaldehyd (Hexanal), 2-Hexenal (β-Propylacrolein), Hydrozimtaldehyd (3-Phenyl-1-propanal), Laurylaldehyd (Docdecanal), Nonylaldehyd (n-Nonanal), Octylaldehyd (n-Octanal), Phenylacetaldehyd (1-Oxo-2-phenylethan), Propionaldehyd (Propanal), Vanillin, Zimtaldehyd (3-Phenylpropenal), Perillaaldehyd und Cuminaldehyd verwendbar.

Erfindungsgemäß einsetzbar sind beispielsweise auch die im folgenden aufgeführten etherischen Öle und/oder die alkoholischen, glykolischen oder durch $CO_2$-Hochdruckverfahren erhaltenen Extrakte aus den genannten Pflanzen (Komponente (h)):

(h1) Öle bzw. Extrakte mit hohem Anteil an Alkoholen: Melisse, Koriander, Kardamon, Eukalyptus;
(h2) Öle bzw. Extrakte mit hohem Anteil an Aldehyden: Eukalyptus citriodora, Zimt, Zitrone, Lemongras, Melisse, Citronella, Limette, Orange;
(h3) Öle bzw. Extrakte mit hohem Anteil an Phenolen: Oreganum, Thymian, Rosmarin, Orange, Nelke, Fenchel, Campher, Mandarine, Anis, Cascarille, Estragon und Piment;
(h4) Öle bzw. Extrakte mit hohem Anteil an Acetaten: Lavendel;
(h5) Öle bzw. Extrakte mit hohem Anteil an Estern: Senf, Zwiebel, Knoblauch;
(h6) Öle bzw. Extrakte mit hohem Anteil an Terpenen: Pfeffer, Pomeranze, Kümmel, Dill, Zitrone, Pfefferminz, Muskatnuß.

Der Anteil der Komponenten (c) - (h) in der Aromastoffkomponente (C) bzw. (C') ist vorzugsweise kleiner oder gleich 25 Gew.-% und liegt bevorzugt im Bereich von 0,001 bis 9 Gew.-%. Bevorzugt unter den weiteren GRAS-Aromastoffen sind die Phenole (c) und etherischen Öle (h).

Besonders bevorzugt im Sinne der vorliegenden Erfindung sind Aromstoffkomponente (C) bzw. (C'), deren antimikrobiell wirksamer Bestandteil ausschließlich aus GRAS-Aromastoffen besteht, d. h. keine "Derivate" der GRAS-Aromastoffe enthält. Als Beispiel einer solchen Zusammensetzung ist ein Gemisch aus Benzylalkohol, einem oder zwei der vorstehend genannten GRAS-Aroma-Alkohole (a2) und Tannin zu nennen. Dieses Gemisch enthält dabei vorzugsweise 0,1 - 99,9, besonders bevorzugt 0,1 - 20 Gew.-% Benzylalkohol und 0,01 - 10 Gew.-% Tannin. Ein weiteres Beispiel einer bevorzugten Zusammensetzung ist ein Gemisch aus 2 Alkoholen, einem Polyphenol (insbesondere Tannin) und einem etherischen Öl (insbesondere einem phenolischen etherischen Öl, Komponente (h3)).

Neben den Komponenten (A) bis (C) können zusätzlich noch weitere Verbindungen (D) wie Alkohole (D1) Emulgatoren (D2), Stabilisatoren (D3), Antioxidantien (D4), Konservierungsmittel (D5), Lösemittel (D6), Trägerstoffe (D7) etc. eingesetzt werden.

Der Anteil der Komponenten (D) an der geruchsmaskierenden Zusammensetzung darf bis 99 Gew.-% betragen, ist vorzugsweise kleiner als 50 Gew.-% und liegt besonders bevorzugt im Bereich von 0,1 bis 20 Gew.-%.

Bei den Alkoholen (D1) handelt es sich erfindungsgemäß um einwertige oder mehrwertige Alkohole mit 2 bis 10 C-Atomen, vorzugsweise mit 2 bis 7 C-Atomen, wobei die GRAS-Alkohole (a) hiervon nicht umfaßt sind. Vorzugsweise werden solche Mengen an GRAS-Aroma-Alkoholen (a) und weiteren Alkoholen (D1) eingesetzt, daß deren Mischungsverhältnis zwischen 1000 : 1 und 1 : 1000, insbesondere zwischen 100 : 1 und 1 : 100 und besonders bevorzugt zwischen 10 : 1 und 1 : 10 liegt.

Bei den Trägerstoffen D7 handelt es sich vorzugsweise um polymere Verbindungen wie Propylenglykol usw.

Es kann bei bestimmten Anwendungen, z.B. wenn die geruchsmaskierende Zusammensetzung mit Lebensmittel in Kontakt kommt oder in von Personen bewohnten Räumen eingesetzt wird, angebracht sein, dass Systeme eingesetzt werden, die frei von Ethanol und Isopropanol bzw. frei von bedenklichen Dosierungen von Ethanol und Isopropanol sind, da diese Stoffe z. B. von Lebensmitteln absorbiert werden können und auch von den Personen in den behandelten Räumen eingeatmet wer den können. Darüber hinaus kann bei der Verwendung dieser Verbindungen Explosionsgefahr bestehen.

Das Verteilen/Zerstäuben der antimikrobiellen Zusammensetzung erfolgt durch handelsübliche Zweistoffdüsen oder Verdampfungstechniken. Hierfür vorgesehene Vorrichtungen, wie eine Bubbler-Einrichtung, die die Luft mit Entkeimungsmittel in feinster Verteilung und niedrigst möglicher Dosierung beaufschlagt, eine speziell für die Verpackung anzuwendende Vorrichtung und ein Dochtsystem, sind in den beiliegenden Figuren abgebildet.

Das Zerstäuben erfolgt dabei durch das in der PCT/EP40/02992 beschriebene kontinuierliche Verfahren zur Anreicherung von Luft mit einem Luftbehandlungsmittel (12), bei welchem das Luftbehandlungsmittel (12) in einer flüssigen Phase in die Luft eingebracht wird und verdampft, wobei der Behandlungsmittelanteil in der Luft pro m$^3$ Luft zwischen 0,1 und 0,00001 ml, vorzugsweise zwischen 0,01 und 0,0001 ml, beträgt sowie der hier beschriebenen Vorrichtung zur Anreicherung von Luft mit einem Luftbehandlungsmittel (12) mit

einer Vorratskammer (10) für flüssiges Luftbehandlungsmittel (12),
einer Verwirbelungskammer (16), der flüssiges Luftbehandlungsmittel (12) zugeführt wird, und
einem Mittel (24) zur Erzeugung eines Luftstroms in der Verwirbelungskammer (16), so dass durch den Luftstrom (30,34) eine Verwirbelung des füssigen Luftbehandlungsmittels (12) erfolgt und aus der Verwirbelungskammer (16) ein Gemisch aus Luft und dampfförmigem Luftbehandlungsmittel (12) austritt.

Bei diesen Verfahren wird das Luftbehandlungsmittel aus einer flüssigen Phase in die Luft eingebracht und verdampft. Bei derartig geringen Mengen an Luftbehandlungsmittel pro m$^3$ Luft ist ein Niederschlag des Luftbehandlungsmittels nicht mehr nachweisbar. Das Verfahren kann daher auch zur Luftbehandlung in Lagerräumen für Lebensmittel eingesetzt werden. Auch in Wartezimmern oder Wohnungen von Allergikern u. dgl. ist der Einsatz des erfindungsgemäßen Verfahrens besonders vorteilhaft, da kein störender Niederschlag an kühlen Fenstern o.dgl. auftritt.

Bereits bei einem Luftbehandlungsmittelanteil von 15 ppt (parts per trillion) konnte eine Reduzierung der Geruchsbelästigung von 100 % im Versuch nachgewiesen werden. Der Luftbehandlungsmittelanteil ist vorzugsweise = 100 ppt und insbesondere = 10 ppt.

Vorzugsweise wird bei dem Verfahren zum Einbringen des Luftbehandlungsmittels in die Luft zuerst das Luftbehandlungsmittel aus einer Vorratskammer einer von Luft durchströmten Verwirbelungskammer zugeführt. Hierbei wird die der Verwirbelungskammer zugeführte Luftmenge und die der Verwirbelungskammer zugeführte Menge an Luftbehandlungsmittel so eingestellt, dass der Luftbehandlungsmittelanteil zwischen 0,1 und 0,00001 ml, vorzugsweise zwischen 0,01 und 0,0001 ml pro m$^3$ Luft pro Stunde, beträgt. Anschließend wird das Gemisch aus Luft und dampfförmigem Luftbehandlungsmittel in den zu behandelnden Raum eingeleitet.

Die Verdampfung des Luftbehandlungsmittels findet hierbei ohne Zufuhr von Wärme statt. Ausschließlich aufgrund der Verwirbelung des Luftbehandlungsmittels wird die Aufnahme der geringen Menge an Luftbehandlungsmittel durch die Luft erreicht. Die von dem Luftstrom mitgerissene Menge an Luftbehandlungsmittel ist so gering, dass kein Aerosol entsteht. Durch die Verwirbelung des Luftbehandlungsmittels in der Verwirbelungskammer wird eine Vielzahl von Luftblasen erzeugt. Hierdurch wird die Oberfläche des Luftbehandlungsmittels derart vergrößert, dass von dem Luftstrom geringe Mengen an Luftbehandlungsmittel aufgenommen werden.

Die Menge an Luft, die der Verwirbelungskammer zugeführt wird, sowie die Menge an Luftbehandlungsmittel, die der Verwirbelungskammer zugeführt wird, kann empirisch ermittelt werden. Hierbei ist darauf zu achten, dass die Geschwindigkeit des Luftstroms nicht so hoch ist, dass Tröpfchen von Luftbehandlungsmittel mitgerissen werden. Andererseits führt eine zu geringe Menge an in der Verwirbelungskammer enthaltenem Luftbehandlungsmittel dazu, dass keine ausreichende Verwirbelung stattfindet. Es wurde herausgefunden, dass besonders gute Ergebnisse bei eine Verhältnis der zugeführten Luftmenge zu der zugeführten Menge an Luftbehandlungsmittel zwischen $\frac{45\%}{55\%}$ und $\frac{30\%}{70\%}$ erzielt werden können. Vorzugsweise liegt dieses Verhältnis zwischen $\frac{42\%}{58\%}$ und $\frac{35\%}{65\%}$.

Vorzugsweise wird das Gemisch aus Luft und Luftbehandlungsmittel vor dem Einleiten in den zu behandelnden Raum durch eine Zwischenkammer geleitet, der durch eine Rückhaltescheibe von der Verwirbelungskammer getrennt ist. Die Zwischenkammer dient dazu, dass zu viel in der Luft enthaltenes Luftbehandlungsmittel auskondensieren kann. Dies wird durch die Rückhaltescheibe, die vorzugsweise feine Öffnung hat oder als feinporige Membran ausgebildet ist, noch unterstützt. Die Zwischenkammer dient somit als Tropfenabscheider. Hierdurch ist sichergestellt, dass kein Aerosol in den zu behandelnden Raum gelangt. Bei dem in den zu behandelnden Raum strömenden Gemisch aus Luft und dampfförmigem Lufthandlungsmittel kann ein Niederschlag mit herkömmlichen Methoden nicht nachgewiesen werden.

Da die in die Verwirbelungskammer eingeleitete Menge an Luftbehandlungsmittel erheblich größer ist als die in dem Gemisch aus Luft und Luftbehandlungsmittel enthaltene Behandlungsmittelanteil wird überschüssiges Lufbehandlungsmittel aus der Verwirbelungskammer abgeführt. Vorzugsweise wird das Luftbehandlungsmittel in die Vorratskammer zurückgeführt. Aus dieser kann es unmittelbar wieder in die Verwirbelungskammer eingeleitet werden.

Die Vorrichtung zur Anreicherung von Luft mit einem Luftbehandlungsmittel, die insbesondere zur Luftentkeimung geeignet ist, weist eine Vorratskammer, eine Verwir belungskammer und ein Mittel zur Erzeugung eines Luftstroms auf. In der Vorratskammer ist flüssiges Luftbehandlungsmittel enthalten. Das flüssige Luftbehandlungsmittel wird beispielsweise mittels einer Pumpe der Verwirbelungskammer zugeführt. Bei dem Mittel zur Erzeugung eines Luftstroms kann es sich je nach Aufbau der Vorrichtung um einen das Gemisch aus der Verwirbelungskammer saugenden Ventilator oder einen Luft in die Verwirbelungskammer blasenden Ventilator handeln. Der Ventilator ist so angeordnet, dass in der Verwirbelungskammer ein Luftstrom entsteht, durch den eine Verwirbelung des flüssige Behandlungsmittels erfolgt. Durch die Verwirbelung des Luftbehandlungsmittels nimmt die Luft eine geringe Menge an Luftbehandlungsmittel auf, so dass aus der Verwirbelungskammer ein Gemisch aus Luft und dampfförmigem Luftbehandlungsmittel austritt.

Die Vorrichtung ist zur Durchführung des erfindungsgemäßen Verfahrens geeignet, so dass das aus der Vorrichtung austretende Gemisch aus Luft und dampfförmigem Luftbehandlungsmittel einen Luftbehandlungsmittelanteil pro m$^3$ Luft pro Stunde zwischen 0,1 und 0,00001 ml, vorzugsweise zwischen 0,01 und 0,0001 ml, aufweist. Je nach Art des Behandlungsmittels kann der Behandlungsmittelanteil in der Luft durch das Verhältnis von zugeführter Luftmenge und zugeführter Menge an Behandlungsmittel zu der Verwirbelungskammer eingestellt werden. Es wurde herausgefunden, dass bei einem Verhältnis von Luftmenge zu Behandlungsmittelmenge zwischen $\frac{45\%}{55\%}$ und $\frac{30\%}{70\%}$, vorzugsweise zwischen $\frac{42\%}{58\%}$ und $\frac{35\%}{65\%}$, ein derart geringer Behandlungsmittelanteil erzielt werden kann.

Vorzugsweise weist die Verwirbelungskammer im Bodenbereich Lufteintrittsöffnungen auf, durch die Luft in die Verwirbelungskammer einströmt. Ferner kann überschüssiges Luftbehandlungsmittel aus der Verwirbelungskammer durch die Lufteintrittsöffnungen in dem Luftstrom entgegengesetzter Richtung ablaufen.

In Versuchen mit einem Luftentkeimungsmittel wurde bei einem Luftdurchsatz von ca. 1100 m$^3$ pro Stunde ein Behandlungsmittelanteil von 0,01 ml pro m$^3$ Luft erzielt. Bei den vorstehend angegebenen Verhältnissen zwischen Luft und Behandlungsmittel wird somit nur ein sehr geringer Anteil an Luftbehandlungsmittel in der Luft aufgenommen und ein Großteil des Luftbehandlungsmittels wird aus der Verwirbelungskammer abgeführt. Hierbei handelt es sich um einen überraschenden Effekt, da trotz der sehr großen Menge an Luftbehandlungsmittel in der Verwirbelungskammer durch die Verwirbelung ein sehr geringer Anteil Luftbehandlungsmittel von der Luft aufgenommen wird. Derart geringe Mengen an Luftbehandlungsmittel in die Luft einzubringen, ist mit Sprühtechniken oder mit Wärmeverdampfung nicht möglich. Dies ist insbesondere nicht möglich, wenn bekannte Vorrichtung ohne Taktung betrieben werden. Bei der erfindungsgemäßen Vorrichtung wurde das vorstehende Ergebnis jedoch ohne Taktung erreicht.

Um sicherzustellen, dass tatsächlich kein sich niederschlagendes Aerosol aus der Vorrichtung entweicht, ist der Verwirbelungskammer eine Zwischenkammer nachgeschaltet. Zwischen der Zwischenkammer und der Verwirbelungskammer ist eine Rückhaltescheibe vorgesehen. Gegebenenfalls von dem Luftstrom mitgerissene Tröpfchen an Luftbehandlungsmittel werden einerseits von der Rückhaltescheibe zurückgehalten und kondensieren andererseits in der Zwischenkammer aus.

Vorzugsweise sind den Lufteintrittsöffnungen der Verwirbelungskammer Filter vorgeschaltet, um eine möglichst keimfrei, partikelfreie und bakterienfreie Luft der Vorrichtung zuzuführen. Hierzu ist ein Partikelfilter und/oder ein Bakterienfilter und/oder ein Feuchtigkeitsfilter vorgesehen.

Vorteilhafterweise wird die Vorrichtung mit einer Klimaanlage gekoppelt, so dass durch die Klimaanlage das Verteilen des Luftbehandlungsmittels im gesamten Raum gewährleistet ist.

Der Vorrichtung kann eine Druckerzeugungseinrichtung nachgeschaltet sein, die den Druck des austretenden Gemisches aus Luft und dampfförmigem Luftbehandlungsmittel erhöht. Eine derartige Vorrichtung kann beispielsweise verwendet werden, um sicherzustellen, dass das Gemisch auch in die Ecken eines Raumes geblasen wird.

An eine Vorrichtung mit angeschlossener Druckerzeugungseinrichtung kann eine Lanze mit Luftaustrittsöffnungen angeschlossen werden. Die Lanze kann in Lebensmittelverpackungen eingeführt werden, um das Luftbehandlungsmittel in die Verpackung einzuleiten.

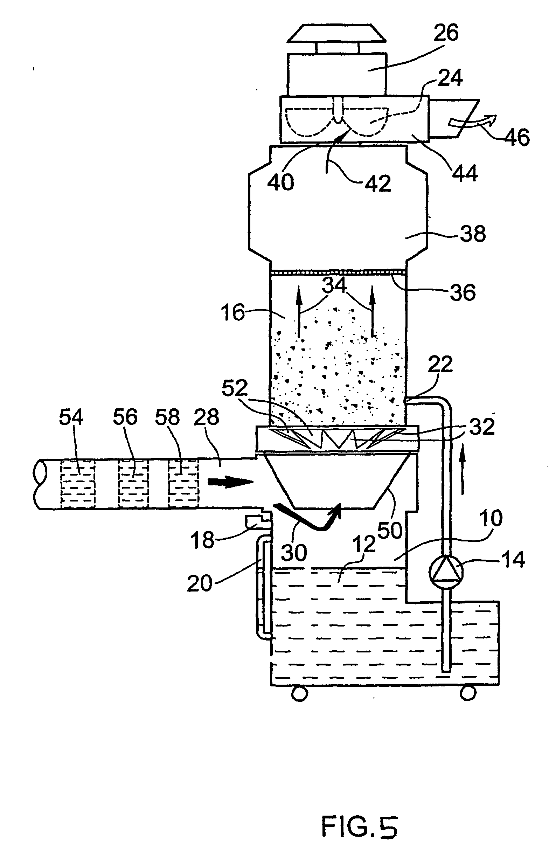

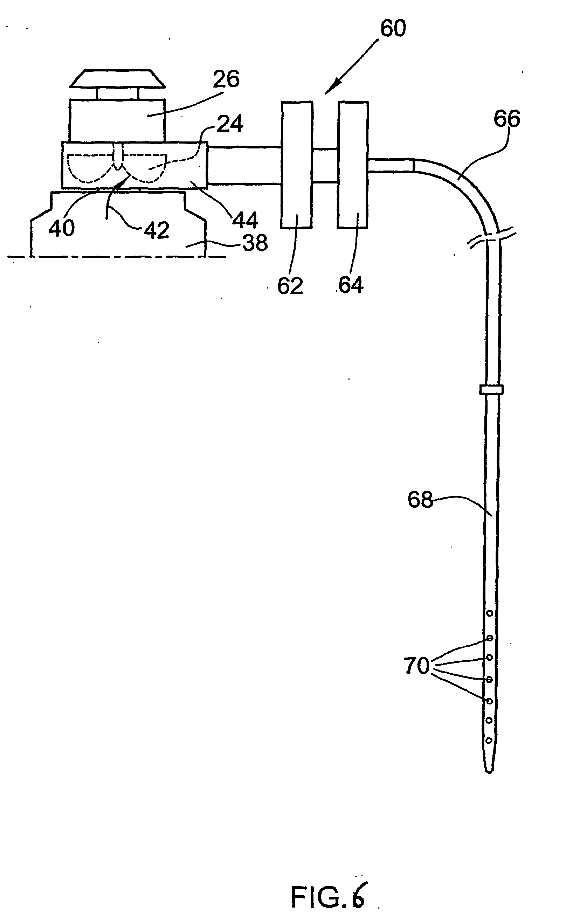

Nachfolgend werden die in Figuren 5 und 6 gezeigten Vorrichtungen näher erläutert, Es zeigen:

In einer Vorratskammer 10 ist Luftbehandlungsmittel 12 enthalten. Das Luftbehandlungsmittel 12 wird mittels einer Pumpe 14 aus der Vorratskammer 10 in eine Verwirbelungskammer 16 gepumpt. Die Vorratskammer 10 ist ferner mit einem Einfüllstutzen 18 zum Nachfüllen von Luftbehandlungsmittel 12 und mit einer Füllstandsanzeige 20 in Form eines durchsichtigen Rohrs versehen.

Das aus der Vorratskammer 10 in die Verwirbelungskammer 16 gepumpte Luftbehandlungsmittel 12 wird über eine Zuführöffnung 22 der Verwirbelungskammer 16 zugeführt. In Abhängigkeit des Pumpendrucks und der Größe der Zuführöffnung 22 wird das Luftbehandlungsmittel 12 mit unterschiedlichem Druck in die Verwirbelungskammer 16 eingespritzt. Durch das Einspritzen des Luftbehandlungsmittels 12 kann der Verwirbelungseffekt in der Verwirbelungskammer 16 erhöht werden.

Mittels eines als Mittel zur Erzeugung eines Luftstroms dienenden Ventilators 24, der von einem Motor 26 angetrieben ist, wird Luft durch einen Luftzuführkanal 28 in den oberen Bereich der Vorratskammer 10 gesaugt. Aus diesem tritt die Luft in Richtung des Pfeils 30 durch im Bodenbereich der Verwirbelungskammer 16 angeordnete Lufteintrittsöffnungen 32 in die Verwirbelungskammer 16 ein. Aus dieser tritt der Luftstrom in Richtung der Pfeile 34 durch eine Rückhaltescheibe 36 hindurch in eine Zwischenkammer 38 ein. Aus der Zwischenkammer 38 tritt das Gemisch aus Luft und Luftbehandlungsmittel durch einen rohrförmigen Ansatzstutzen 40 in Richtung des Pfeils 42 in einen Ventilatorraum 44 und aus diesem in Richtung eines Pfeils 46 in den zu behandelnden Raum ein.

Die im Bodenbereich der Verwirbelungskammer 16 vorgesehenen Lufteintrittsöffnungen 32 sind sternförmig angeordnete Schlitze, durch die die Luft in die Verwirbelungskammer 16 eintritt. Da die der Verwirbelungskammer 16 zugeführte Menge an Luftbehandlungsmittel 12 größer ist als der Luftbehandlungsmittelanteil in dem aus der Vorrichtung austretenden Gemisch, muß ein Großteil des Luftbehandlungsmittels 12 aus der Verwirbelungskammer 16 wieder in die Vorratskammer 10 zurückgeführt werden. Bei der dargestellten Ausführungsform fließt das überschüssige Luftbehandlungsmittel 12 durch die schlitzförmigen Lufteintrittsöffnungen 32 in die Vorratskammer 10 zurück. Hierzu ist der Bodenbereich der Verwirbelungskammer 16, in der die Lufteintrittsöffnungen 32 vorgesehen sind, trichterförmig ausgebildet.

Um ein gezieltes Zurückfließen des überflüssigen Luftbehandlungsmittels zu gewährleisten, ist im oberen Bereich der Vorratskammer 10 ein Trichter 50 vorgesehen. Durch den Trichter 50 ist ferner verhindert, dass Luftbehandlungsmittel 12 in den Luftzuführkanal 28 gelangt.

Die Schlitzbreite der Lufteintrittsöffnungen 32 ist einstellbar, da der Bodenbereich aus einzelnen dreieckförmigen Segmenten 52 besteht, deren Neigungswinkel verstellbar ist. Je steiler die Segmente 52 angeordnet sind, desto größer sind die schlitzförmigen Lufteintrittsöffnungen 32.

Das aus der Verwirbelungskammer 16 austretende Gemisch aus Luft und Luftbehandlungsmittel wird durch die Rückhaltescheibe 36 hindurch in die Zwischenkammer 38 geführt. Die Rückhaltescheibe 36 weist Öffnungen mit geringem Durchmesser auf oder besteht aus einer feinporigen Membran. Durch die Rückhaltescheibe 36 werden ggf. von dem Luftstrom mitgerissene Luftbehandlungsmittel-Tröpfchen zurückgehalten, so dass möglichst nur dampfförmiges Luftbehandlungsmittel in die Zwischenkammer 38 gelangt.

Die Zwischenkammer 38 ist als zusätzliche Sicherheit vorgesehen. Hierbei ist sichergestellt, dass ggf. in dem Gemisch aus Luft und Luftbehandlungsmittel befindliches Luftbehandlungsmittel, das nicht in dampfförmiger Form vorliegt, in der Zwischenkammer 38 auskondensiert. Der an den Wänden der Zwischenkammer 38 auskondensierende Teil des Luftbehandlungsmittels fließt durch die Rückhaltescheibe 36 zurück in die Verwirbelungskammer 16. Aus der Zwischenkammer 38 tritt entlang des Pfeils 42 ausschließlich ein Gemisch aus Luft und dampfförmigem Luftbehandlungsmittel in die Ventilatorkammer 44 ein. Das in den Ventilatorraum 44 eintretende Gemisch weist kein Aerosol mehr auf, so dass die geringe Menge an Luftbehandlungsmittel, die sich in dem Gemisch befindet, nicht mehr als Niederschlag nachweisbar ist.

In dem Luftzuführkanal 28 ist zum Filtern der angesaugten Luft ein Partikelfilter 54, insbesondere ein Pollenfilter, ein Bakterienfilter 56 und ein Feuchtigkeitsfilter 58 vorgesehen. Durch den Feuchtigkeitsfilter 58 wird die Feuchtigkeit aus der angesaugten Luft entnommen, da die verwendeten Luftbehandlungsmittel häufig hydroskopisch sind.

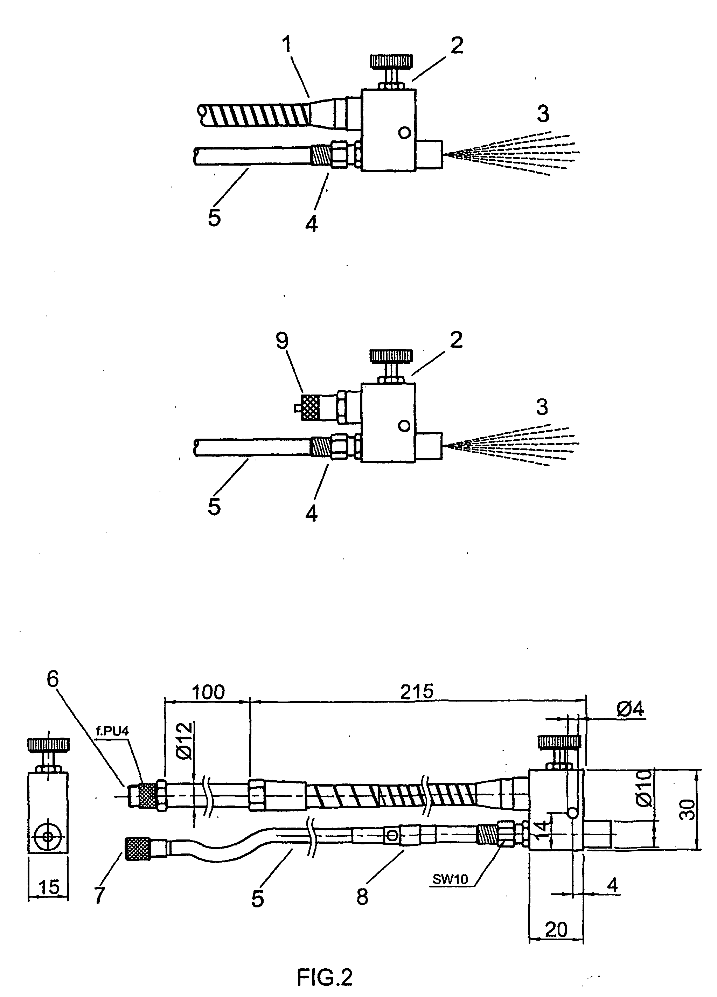

An die Ventilatorkammer 44 kann eine Druckerzeugungseinrichtung 60 (Fig. 2) angeschlossen sein. Im dargestellten Ausführungsbeispiel handelt es sich um eine zweistufige Druckerzeugungseinrichtung mit einer ersten Druckerzeugungsstufe 62 und einer zweiten Druckerzeugungsstufe 64. Nach der Druckerzeugungseinrichtung 60 wird das Gemisch aus Luft und Luftbehandlungsmittel in einen flexiblen Schlauch 66 mit erhöhtem Druck eingeleitet. An den flexiblen Schlauch 66 ist eine Lanze 68 mit Austrittsöffnungen 70 angeschlossen. Die Lanze 68 kann in Lebensmittelverpackungen eingeführt werden, um diese mit dem Gemisch aus Luft und Luftbehandlungsmittel zu füllen.

Das Zerstäuben kann ebenfalls durch ein diskontinuierliches Verfahren, wie in einer parallelen deutschen Gebrauschmusteranmeldung beschrieben (internes Aktenzeichen 003220de), erfolgen.

Das Zerstäuben/Verteilen erfolgt bei dem erfindungsgemäßen Verfahren so, dass die Konzentration der geruchsmaskierenden Zusammensetzung 0,001 bis 1 ml pro $m^3$ Luft, insbesondere 0,01 bis 0,1 ml pro $m^3$ Luft, beträgt. Bei austauschenden Luftsystemen, bei denen eine stündliche Umwälzung erfolgt, ist das Verfahren so einzustellen, daß eine Dosierung von 0,00001 bis 1 ml pro $m^3$ pro Stunde, insbesondere von 0,002 bis 0,02 ml pro $m^3$ pro Stunde, gegeben ist.

Das vorliegende Verfahren eignet sich dadurch sowohl zur Reduzierung der Geruchsbelästigung der Luft in privaten Haushalten, Büros und öffentlichen Gebäuden als auch in lebensmittelverarbeitenden Betrieben, Transportvorrichtungen, Kühl-, Klima- und sonstigen Lüftungsbereichen. Insbesondere wird durch die Verwendung von antimikrobiellen Aromastoffkomponenten zusätzlich eine Entkeimung der Umgebungsluft erzielt, was den Wirkungsgrad des Geruchsreduzierungsverfahrens weiter erhöht.

Die vorliegende Erfindung wird anhand der nachfolgenden Beispiele näher erläutert.

**Beispiele**

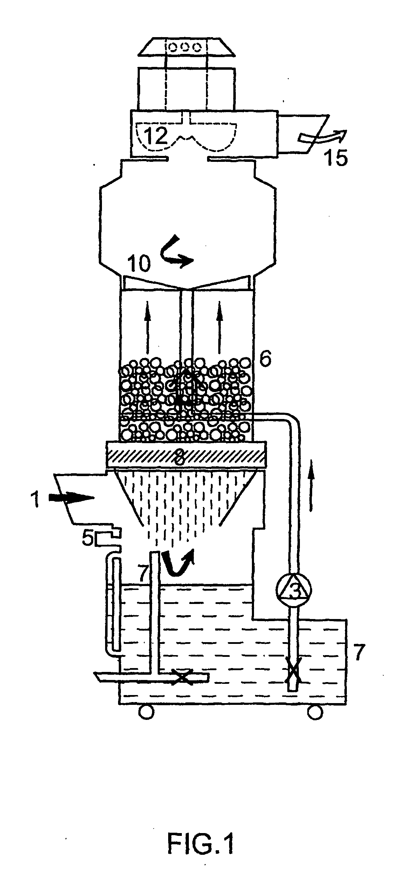

Verwendete Apparaturen: Für die nachfolgend beschriebenen Beispiele wurden die in den Figuren 1 bis 7 abgebildeten Vorrichtungen verwendet.

Fig. 1: Luft-EvL(Entkeimung von Luft)-Bubbler

Autonome, fest installierte oder mobile Bubblereinheit mit eingebautem Abluftventilator und Pumpe. Luftmenge 2 - 1600 $m^3$/h (oder größer)
Funktionsprinzip: Bubbler mit schwebendem EvL-Fließbett
Luft mit gegenströmendem EvL-Mittel. Hier wird EvL-Mittel in einer Kammer mit starkem Unterdruck zum Schweben gebracht. Dadurch entsteht ein Gleichgewicht zwischen Luftunterdruck und EvL-Mittelgewicht. Die Luft verteilt sich auf die gesamte EvL-Fläche und steigt als mikroskopisch kleine Blasen durch das EvL-Bett. Die Luftblasen bilden eine sehr große Kontaktfläche zwischen Gas und Flüssigkeit. Luftdruck und Verweilzeit stehen in einem ausgewogenen Verhältnis. EvL-Mittel wird mit der Luft entsprechend dosiert mittransportiert.

### Ventilator

Der Abluft-Radialventilator befindet sich immer im Reinluftbereich und kann auch extern installiert sein.

### Bubbler

Der Wäscher besteht aus:

- Absorptionsflüssigkeits-Behälter.
- Waschkammer
- Trockenkammer
- Ventilator

### Legende zur Fig. 1

1) Luftansaugstutzen mit/ohne Mikrofilter
2) EvL-Mittelzufuhr
3) z. B. Pumpe 15 m$^3$/h
Motor 220/380 V; 2800 U/min; 1,1 kW
5) Dosiereinheit (elektr.) Menge/Luft Verhältnis EvL-Mitteldosierung 0,02 ml - 0,1 ml/m$^3$ (h) Dosierung
6) EvL-Mittel
7) EvL-Mittel
9) Waschkammer
10) Trockner
12) Ventilator 1200/1800 m$^3$/h
Motor 220/380 V; 2800 U/min; 1,1 kW
15) Ausblasstutzen z.B. $\varnothing$ 200 mm

### Fig. 2: EvL-Zerstäuber-Niederdruck-System (für dünne Flüssigkeiten)

Zum Zerstäuben dünner Öle und Flüssigkeiten mit gezieltem Wirkungsbereich.
Bereits ab 2 bar Überdruck spricht der Zerstäuber an. Der Zerstäuber läßt sich durch den biegsamen Metallschlauch ganz nach Wunsch drehen und wenden und kann mit dem Magnethalter an jeder beliebigen Stelle befestigt werden.

### Funktion:

Bei anstehender Druckluft wird sofort zerstäubt (ein eingebautes Rückschlagventil läßt sdie Flüssigkeit im Schlauch nicht absinken. Der Zerstäuber arbeitet dauernd oder mit dem Blasautomat taktweise - aber immer in wohldosierten Mengen. Im Zentrum des Luftstrahls wird die Flüssigkeit wirtschaftlich und sauber zugeführt. Über die Luft- und Flüssigkeitsdrossel kann die Luft- und Flüssigkeitsmenge fein eingestellt werden. Der Zerstäuber ist von 10° bis 30° Sprühwinkel stufenlos einstellbar.

### Legende zur Fig. 2

1) Metallschlauch vernickelt
2) Luftdrossel
3) Sprühwinkel 10° - 30°
4) Flüssigkeitsdrossel
5) PVC-Schlauch 1 m
6) Anschluß für PK4
7) Slebventil
8) Rückschlagventil
9) Anschluß für Druckluft
10) Drosselkugel (nicht sichtbar)

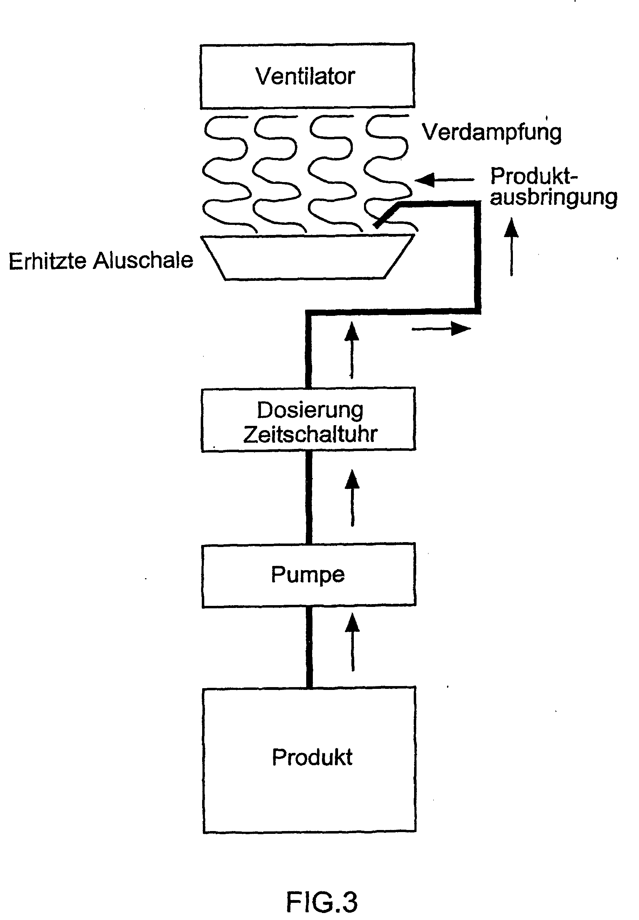

### Fig. 3: EvL-Verdampfungssystem

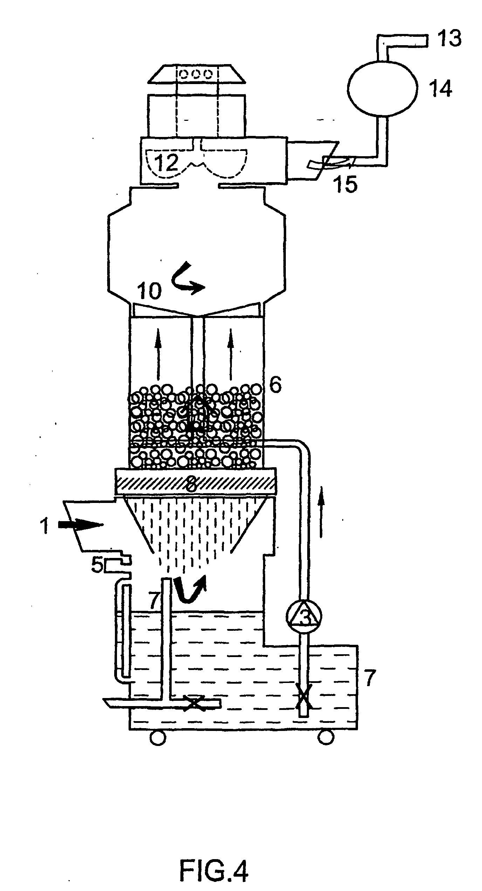

### Fig. 4: EvL-Entkeimung in der Verpackung mit Bubbler

Ventilator: Der Abluft-Radialventilator befindet sich immer im Reinluftbereich und kann auch extern installiert sein.

### Legende zur Fig. 4

1) Luft und/oder CO$_2$/oder Stickstoff o. ä. Ansaugstutzen mit/ohne Mikrofilter
2) EvL-Mittelzufuhr
3) Pumpe 15 m$^3$/h
Motor 220/380 V; 2800 U/min; 1,1 kW
5) Dosiereinheit (elektr.) Menge/Luft Verhältnis EvL-Mitteldosierung 0,02 ml - 0,1 ml/m$^3$ (h) Dosierung
6) EvL-Mittel
7) EvL-Mittel
9) Waschkammer
10) Trockner
12) Ventilator 1200/1800 m$^3$/h
Motor 220/380 V; 2800 U/min; 1,1 kW
13) Ausbringung in die Verpackung (z. B. über Lanze)
14) Druckreservoir (ca. 2 - 8 bar komprimiert) bestehend aus Luft und Co$_2$ und N$_2$ und EvL-Mittel mit geringer Feuchtigkeit
15) Ausblasstutzen z.B. $\varnothing$ 200 mm

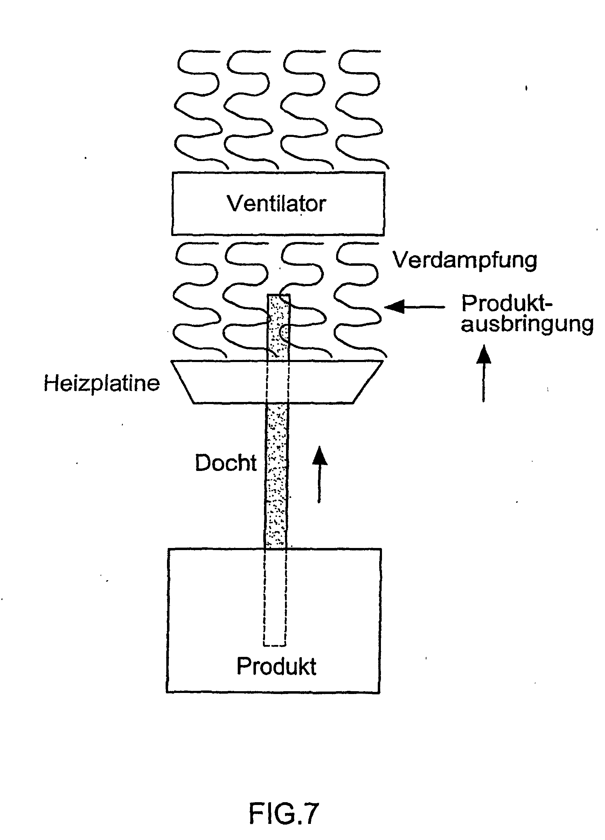

Fig. 7: EvL-Docht-System mit Heizplatine und Ventilator

Beispiel 1: Untersuchung der Geruchsreduzierung mittels der in Figur 1 dargestellten Vorrichtung

Dem Entkeimungsmittel, bestehend aus 1 Gew.-% Polyphenol (Tannin), 3 Gew.-% Benzylalkohol, 0,1 Gew.-% etherisches Öl (phenolisch) und 90,9 Gew.-% Propylenglykol wurde 5 Gew.-% Polyvinylpyrrolidon (Pharmakopöen: Ph. Eur//USP/NF//JP/JPE), Viskosität (20 Gew.-% in Wasser) 20 mPa•s, Molgewicht 35.000 - 50.000) beigemischt und als Prozessmittel in die Dosiervorrichtung des Luft-Evl-Bubblers nach Fig. 1 eingegeben.
Es wurde ein 12,18 m$^2$ geschlossener Raum mit Standarddeckenhöhe ohne Abluft/Zuluft mit Zigarrenasche aus gebrauchten Zigarrenstummeln (34,39 g) kontaminiert in der Weise, dass die Menge auf vier Petrischalen am Boden in den Raumecken verteilt wurde. Die Einwirkzeit bis zur Geruchsoptimierung des Zigarren-Asche-Gestanks betrug 24 h bei 25 °C (geschlossen).
Das Bubblergerät wurde am gleichen Tag ohne Betrieb bereits eingebracht, um spätere Luftverwandlungen zu vermeiden. Am nächsten Tag (EZ: 24 h) nach positiver Geruchsbeurteilung ("intensiver Zigarrengeruch") wurde der Bubbler 24 h in Betrieb gesetzt mit einer Dosierung des Mittels von 0,00159 g/m$^3$ pro Stunde.
Nach 24 Stunden wurde der Raum geöffnet. Er war geruchs- und nach biologischer Luftkeimprobennahme keimfrei. Versuche mit gleichen Dosierungen und Bedingungen des Geruchsneutralisierungsmittels für Luft (Polyvinylpyrrolidon) 5 Gew.-% ad 100 Gew.-% in Wasser und/oder Propylenglykol (ohne Entkeimungsmittel) führten zu gleichen Geruchsneutralisierungs-Resultaten.

**Patentansprüche**

1. Verfahren zur untoxischen Geruchsreduzierung, umfassend das Verteilen oder Zerstäuben einer geruchsmaskierenden Zusammensetzung, weiche als geruchsmaskierende Komponente (A) wenigstens Polyvinylpyrrolidon enthält, in der zu behandelnden Umgebung, wobei die geruchsmaskierende Zusammensetzung wenigstens
eine geruchsmaskierende Komponente (A) ausgewählt aus Maisstärke, Mangansalzen und Polyvinylpyrrolidon, und
eine Aromastoffkomponente (C), ausgewählt aus ätherischen Ölen, Aromastoffen und Duftstoffen enthält,
wobei jedoch die Aromastoffkomponente (C) wenigstens den GRAS(Generally Recognized As Safe in Food)-Aromastoff (gemäß der FEMA/FDA GRAS-Flavour-Substances-Liste 3 - 15 Nr. 2001 - 3905 (Stand 2000)) Benzylalkohol enthält.

2. Verfahren nach Anspruch 1, wobei das Polyvinylpyrrolidon eine Molmasse von 10.000 bis 60.000, bevorzugt von 30.000 bis 50.000, aufweist.

3. Verfahren nach Anspruch 1 oder 2, wobei der Anteil der geruchsmaskierenden Komponente (A) 0,001 bis 50 Gew.-%, vorzugsweise 0,1 bis 20 Gew.-%, der geruchsmaskierenden Zusammensetzung beträgt.

4. Verfahren nach einem oder mehreren der Ansprüche 1 bis 3, wobei die geruchsmaskierende Zusammensetzung weiterhin eine Functional-Flavour-Komponente (B) enthält, die vorzugsweise eine oder besonders bevorzugt mehrere der folgenden Substanzen enthält: Hexylbutyrat, Octylacetat, Isobutylisobutyrat, cis-3-Hexen-1-ylacetat, γ-Decalacton, Ethylcaproat, Butylacetat, Ethylbenzoat, Ethylbutyrat, Hexylacetat, Methylcaproat, Phenylethylalkohol, Citronellol, Undecylaldehyd, Benzylphenylacetat, Cinnamik-Alkohol, Eugenol, Benzylacetat, Linalool, cis-Jasmone, Acetylmethylanthranilat, cis-3-Hexen-1-ol, cis-3-Hexen-1ylsalicylat, Methylbenzoat, Methylsalicylat, Geranylacetat, cis-3-Hexen-1ylacetat, Litsea Cubeba, Phenylpropylalkohol und Phenylethylacetat.

5. Verfahren nach Anspruch 4, wobei der Anteil der Functional-Flavour-Komponente (B) 0,001 bis 20 Gew.-%, vorzugsweise 0,1 bis 5 Gew.-%, der geruchsmaskierenden Zusammensetzung beträgt.

6. Verfahren nach einem oder mehreren der Ansprüche 1 bis 5, wobei der Anteil der Aromastoffkomponente (C) an der geruchsmaskierenden Zusammensetzung 0,001 bis 95 Gew.-%, vorzugsweise 0,1 bis 80 Gew.-%, beträgt.

7. Verfahren nach einem oder mehreren der Ansprüche 1 bis 6 , wobei die Aromastoffkomponente (C) wenigstens zwei GRAS-Aromastoffe enthält und insbesondere einen aromatischen GRAS-Aroma-Alkohol und/oder eine GRAS-Polyphenolverbindung enthält.

8. Verfahren nach einem oder mehreren der Ansprüche 1 bis 7, wobei die Aromastoffkomponente (C)

(a) eine oder mehrere GRAS-Aroma-Alkohole oder deren Derivate und
(b) einen oder mehrere Aromastoffe, ausgewählt aus

(b1) Polyphenolverbindungen und
(b2) GRAS-Aromasäuren oder deren Derivate,

enthält, wobei die Aromastoffkomponente (C) vorzugsweise

> 0,1 bis 99 Gew.-%, vorzugsweise 0,5 bis 99 Gew.-%, Komponente (a),
> 0 bis 25 Gew.-%, vorzugsweise 0,01 bis 10 Gew.-%, Komponente (b1) und
> 0 bis 70 Gew.-%, vorzugsweise 0,01 bis 30 Gew.-%, Komponente (b2)

enthält.

9. Verfahren nach Anspruch 8, wobei der GRAS-Aroma-Alkohol (a) ausgewählt ist aus:

> Benzylalkohol, Acetoin, Ethylalkohol, Propylalkohol, iso-Propylalkohol, Propylenglykol, Glycerin, n-Butylalkohol, iso-Butylalkohol, Hexylalkohol, L-Menthol, Octylalkohol, Zimtalkohol, $\alpha$-Methylbenzylalkohol, Heptylalkohol, n-Amylalkohol, iso-Amylalkohol, Anisalkohol, Citronellol, n-Decylalkohol, Geraniol, $\beta$-$\gamma$-Hexanol, Laurylalkohol, Linalool, Nerolidol, Nonadienol, Nonylalkohol, Rhodinol, Terpineol, Borneol, Clineol, Anisol, Cuminylalkohol, 10-Undecen-1-ol, 1-Hexadecanol oder deren Derivate,

die Polyphenolverbindung (b1) ausgewählt ist aus:

> Brenzcatechin, Resorcin, Hydrochinon, Phloroglucin, Pyrogallol, Cyclohexan, Usninsäure, Acylpolyphenolen, Ligninen, Anthocyane, Flavonen, Catechinen, Gallussäurederivaten, Kaffesäure, Flavonoiden, Derivaten der genannten Polyphenole und Extrakten aus Camellia Primula und

die GRAS-Säure (b2) ausgewählt ist aus:

> Essigsäure, Aconitsäure, Adipinsäure, Ameisensäure, Apfelsäure, Capronsäure, Hydrozimtsäure, Pelargonsäure, Milchsäure, Phenoxyessigsäure, Phenylessigsäure, Valeriansäure, iso-Valeriansäure, Zimtsäure, Citronensäure, Mandelsäure, Weinsäure, Fumarsäure, Tanninsäure und deren Derivate.

10. Verfahren nach einem oder mehreren der Ansprüche 7 bis 9, wobei die Aromastoffkomponente

> (a1) einen.aromatischen GRAS-Aroma-Alkohol, insbesondere Benzylalkohol, als notwendigen Bestandteil und gegebenenfalls
> (a2) einen oder mehrere weitere GRAS-Aroma-Alkohole oder deren Derivate und
> (b1) eine oder mehrere Polyphenolverbindungen und/oder
> (b2) eine oder mehrere GRAS-Säuren oder deren Derivate

enthält, wobei die Aromastoffkomponente vorzugsweise

> 0,1 bis 99 Gew.-%, vorzugsweise 0,1 bis 75 Gew.-% Benzylalkohol;
> 0 bis 99,8 Gew.-%, vorzugsweise 0,01 bis 99 Gew.-% Komponente (a2); und
> 0 bis 25 Gew.-%, vorzugsweise 0,01 bis 10 Gew.-% Komponente (b1),
> 0 bis 70 Gew.-%, vorzugsweise 0,01 bis 30 Gew.-% Komponente (b2)

enthält.

11. Verfahren nach einem oder mehreren der Ansprüche 7 bis 10, wobei die Aromastoffkomponente noch weitere GRAS-Aromastoffe, ausgewählt aus (c) Phenolen, (d) Estern, (e) Terpenen, (f) Acetalen, (g) Aldehyden und (h) etherischen Ölen, enthält, wobei die Aromastoffkomponente vorzugsweise 0,001 bis 25 Gew.-%, vorzugsweise 0,01 bis 9 Gew.-%, der weiteren GRAS-Aromastoffe (c) - (h) enthält.

12. Verfahren nach Anspruch 11, wobei die weiteren GRAS-Aromastoffe Phenole (c) und/oder etherische Öle (h) sind.

13. Verfahren nach einem oder mehreren der Ansprüche 8 bis 12, wobei die Aromastoffkomponente keine Derivate der GRAS-Aromastoffe enthält.

14. Verfahren nach einem oder mehreren der Ansprüche 10 bis 13, wobei die Aromastoffkomponente ein oder zwei GRAS-Aroma-Alkohole (a2) und wenigstens eine Polyphenolverbindung (b1) enthält, wobei die Polyphenolverbindung (b1) vorzugsweise Tannin ist.

15. Verfahren nach Anspruch 14, wobei die Aromastoffkomponente 0,1 - 20 Gew.-% Benzylalkohol und 0,01-10 Gew.-% Tannin enthält.

16. Verfahren nach einem oder mehreren der Ansprüche 1 und 7 bis 15, wobei die Zusammensetzung weiterhin ein- oder mehrwertige Alkohole mit 2 bis 10 C-Atomen, Emulgatoren, Stabilisatoren, Antioxidantien, Konservierungsmittel, Lösemittel und/oder Trägerstoffe enthält.

17. Verfahren nach einem oder mehreren der Ansprüche 7 bis 16, wobei das Zerstäuben der geruchsmaskierenden Zusammensetzung in der Luft durch ein Zweistoffdüsensystem, Verdampfungssystem, eine Bubbleranlage oder ein Dochtsystem erfolgt, wobei vorzugsweise

> (i) in dem der zu behandelnden Umgebung zugeführten Gemisch aus Luft und geruchsmaskierender Zusammensetzung der Anteil an geruchsmaskierender Zusammensetzung 10 bis 100 ppt beträgt; oder

(ii) durch das Verteilen oder Zerstäuben der geruchsmaskierenden Zusammensetzung eine Dosierung von 0,000001 bis 1 ml pro m$^3$ Luft pro Stunde, vorzugsweise von 0,001 bis 0,1 ml pro m$^3$ Luft pro Stunde erzielt wird.

18. Geruchsmaskierende Zusammensetzung zur untoxischen Geruchsreduzierung, wie in Ansprüchen 1 bis 16 definiert,
wobei die geruchsmaskierende Zusammensetzung wenigstens
eine geruchsmaskierende Komponente (A) ausgewählt aus Maisstärke, Mangansalzen und Polyvinylpyrrolidon, wobei die geruchsmaskierende Zusammensetzung jedoch wenigstens Polyvinylpyrrolidon enthält, und
eine Arömastoffkomponente (C), ausgewählt aus ätherischen Ölen, Aromastoffen und Duftstoffen enthält,
wobei jedoch die Aromastoffkomponente (C) wenigstens den GRAS(Generally Recognized As Safe in Food)-Aromastoff (gemäß der FEMA/FDA GRAS-Flavour-Substances-Liste 3 - 15 Nr. 2001 - 3905 (Stand 2000)) Benzylalkohol enthält, vorausgesetzt dass die Zusammensetzung, wenn sie 0,5 - 5,0 Gew.-% Polyvinylpyrrolidon enthält, nicht 0,1 - 10,0 Gew.-% einer Naphthalinsulfonsäure und/ oder deren Salze enthält.

19. Verwendung einer geruchsmaskierenden Zusammensetzung, wie in Anspruch 18 definiert, zur untoxischen Geruchsreduzierung.

**Claims**

1. A method for non-toxic odor reduction, comprising the distributing or atomizing of an odor-masking composition which contains at least polyvinylpyrrolidone as an odor-masking component (A) in the environment to be treated, wherein said odor-masking composition contains at least one odormasking component (A) selected from corn starch, manganese salts and polyvinylpyrrolidone, and a flavor component (C) selected from essential oils, flavoring agents and fragrances, but wherein said flavor component (C) contains at least benzyl alcohol as a GRAS (generally recognized as safe in food) flavoring agent (according to the FEMA/FDA GRAS Flavour Substances List 3-15 Nos. 2001-3905 (as of 2000)).

2. The method according to claim 1, wherein said polyvinylpyrrolidone has a molecular weight of from 10,000 to 60,000, preferably from 30,000 to 50,000.

3. The method according to claim 1 or 2, wherein the proportion of said odor-masking component (A) is from 0.001 to 50% by weight, preferably from 0.1 to 20% by weight, of the odor-masking composition.

4. The method according to one or more of claims 1 to 3, wherein said odor-masking composition further contains a functional flavor component (B) which preferably contains one or several, more preferably several, of the following substances:

hexyl butyrate, octyl acetate, isobutyl isobutyrate, cis-3-hexene-1-yl acetate, γ-decalactone, ethyl caproate, butyl acetate, ethyl benzoate, ethyl butyrate, hexyl acetate, methyl caproate, phenylethyl alcohol, citronellol, undecyl aldehyde, benzylphenyl acetate, cinnamyl alcohol, eugenol, benzyl acetate, linalool, cis-jasmone, acetylmethyl anthranilate, cis-3-hexene-1-ol, cis-3-hexene-1-yl salicylate, methyl benzoate, methyl salicylate, geranyl acetate, cis-3-hexene-1-yl acetate, Litsea cubeba, phenylpropyl alcohol and phenylethyl acetate.

5. The method according to claim 4, wherein the proportion of said functional flavor component (B) is from 0.001 to 20% by weight, preferably from 0.1 to 5% by weight, of said odor-masking composition.

6. The method according to one or more of claims 1 to 5, wherein the proportion of said flavor component (C) in said odor-masking composition is from 0.001 to 95% by weight, preferably from 0.1 to 80% by weight.

7. The method according to one or more of claims 1 to 6, wherein said flavor component (C) contains two GRAS flavoring agents, especially an aromatic GRAS flavor alcohol and/or a GRAS polyphenol compound.

8. The method according to one or more of claims 1 to 7, wherein said flavor component (C) contains:

(a) one or more GRAS flavor alcohols or derivatives thereof; and
(b) one or more flavoring agents selected from

(b1) polyphenol compounds; and
(b2) GRAS flavor acids or derivatives thereof;

wherein said flavor component (C) preferably contains:

from 0.1 to 99% by weight, preferably from 0.5 to 99% by weight, of component (a);
from 0 to 25% by weight, preferably from 0.01 to 10% by weight, of component (b1); and
from 0 to 70% by weight, preferably from 0.01 to 30% by weight, of component (b2).

9. The method according to claim 8, wherein said GRAS flavor alcohol (a) is selected from:

benzyl alcohol, acetoin, ethyl alcohol, propyl alcohol, iso-propyl alcohol, propylene glycol, glycerol, n-butyl alcohol, iso-butyl alcohol, hexyl alcohol, L-menthol, octyl alcohol, cinnamyl alcohol, α-methylbenzyl alcohol, heptyl alcohol, n-amyl alcohol, iso-amyl alcohol, anisalcohol, citronellol, n-decyl alcohol, geraniol, β-γ-hexenol, lauryl alcohol, linalool, nerolidol, nonadienol, nonyl alcohol, rhodinol, terpineol, borneol, clineol, anisole, cuminyl alcohol, 10-undecene-1-ol, 1-hexadecanol, or derivatives thereof;

said polyphenol compound (b1) is selected from:

catechol, resorcinol, hydroquinone, phloroglucinol, pyrogallol, cyclohexane, usnic acid, acylpolyphenols, lignins, anthocyans, flavones, catechols, gallic acid derivatives, caffeic acid, flavonoids, derivatives of the mentioned polyphenols, and extracts from Camellia, Primula; and

said GRAS acid (b2) is selected from:

acetic acid, aconitic acid, adipic acid, formic acid, malic acid, capronic acid, hydrocinnamic acid, pelargonic acid, lactic acid, phenoxyacetic acid, phenylacetic acid, valeric acid, iso-valeric acid, cinnamic acid, citric acid, mandelic acid, tartaric acid, fumaric acid, tannic acid and derivatives thereof.

10. The method according to one or more of claims 7 to 9, wherein said flavor component contains:

(a1) an aromatic GRAS alcohol, especially benzyl alcohol, as a necessary component; and optionally
(a2) one or more further GRAS flavor alcohols or derivatives thereof; and
(b1) one or more polyphenol compounds; and/or
(b2) one or more GRAS acids or derivatives thereof;

wherein said flavor component preferably contains:

from 0.1 to 99% by weight, preferably from 0.1 to 75% by weight, of benzyl alcohol;
from 0 to 99.8% by weight, preferably from 0.01 to 99% by weight, of component (a2); and
from 0 to 25% by weight, preferably from 0.01 to 10% by weight, of component (b1);
from 0 to 70% by weight, preferably from 0.01 to 30% by weight, of component (b2).

11. The method according to one or more of claims 7 to 10, wherein said flavor component contains further GRAS flavoring agents selected from (c) phenols, (d) esters, (e) terpenes, (f) acetals, (g) aldehydes and (h) essential oils;
wherein said flavor component preferably contains from 0.001 to 25% by weight, preferably from 0.01 to 9% by weight, of said further GRAS flavoring agents (c) to (h).

12. The method according to claim 11, wherein said further GRAS flavor agents are phenols (c) and/or essential oils (h).

13. The method according to one or more of claims 8 to 12, wherein said flavor component does not contain any derivatives of the GRAS flavoring agents.

14. The method according to one or more of claims 10 to 13, wherein said flavor component contains one or two GRAS flavor alcohols (a2) and at least one polyphenol compound (b1), wherein said polyphenol compound (b1) is preferably tannin.

15. The method according to claim 14, wherein said flavor component contains from 0.1 to 20% by weight of benzyl alcohol and from 0.01 to 10% by weight of tannin.

16. The method according to one or more of claims 1 and 7 to 15, wherein said composition further contains monohydric or polyhydric alcohols having from 2 to 10 carbon atoms, emulsifiers, stabilizers, antioxidants, preservatives, solvents and/or carriers.

17. The method according to one or more of claims 7 to 16, wherein said atomizing of said odor-masking composition in the air is effected by a two-fluid nozzle system, evaporation system, bubbler device or wick system,
wherein preferably

(i) the proportion of odor-masking composition in the mixture of air and odor-masking composition supplied to the environment to be treated is from 10 to 100 ppt; or

(ii) a dosage of from 0.000001 to 1 ml per m$^3$

of air per hour, preferably from 0.001 to 0.1 ml per $m^3$ of air per hour, is achieved by said distributing or atomizing of said odor-masking composition.

18. An odor-masking composition for non-toxic odor reduction as defined in claims 1 to 16, wherein said odor-masking composition contains at least one odor-masking component (A) selected from corn starch, manganese salts and polyvinylpyrrolidone, provided that said odor-masking composition contains at least polyvinylpyrrolidone, and a flavor component (C) selected from essential oils, flavoring agents and fragrances, but wherein said flavor component (C) contains at least benzyl alcohol as a GRAS (generally recognized as safe in food) flavoring agent (according to the FEMA/FDA GRAS Flavour Substances List 3-15 Nos. 2001-3905 (as of 2000)), provided that if the composition contains from 0.5 to 5.0% by weight of polyvinylpyrrolidone, it does not contain from 0.1 to 10.0% by weight of a naphthalenesulfonic acid and/or its salts.

19. Use of an odor-masking composition as defined in claim 18 for non-toxic odor reduction.


**Revendications**

1. Procédé de réduction d'odeurs non toxiques, comprenant la distribution ou la pulvérisation dans la zone à traiter d'une composition masquant les odeurs, laquelle contient à titre de composant masquant les odeurs (A) au moins une polyvinyl-pyrrolidone, ladite composition masquant les odeurs contenant au moins :

   - un composant masquant les odeurs (A) choisi dans le groupe comprenant amidon de mais, sels de manganèse et polyvinyl-pyrrolidone, et
   - un composant à base de matière aromatique (C) choisie dans le groupe comprenant les huiles éthérées, les constituants aromatiques et les parfums,

   le composant à base de matière aromatique (C), contenant cependant au moins le constituant aromatique dit GRAS {Généralement Considéré Comme Sûr selon «Flavour Substances GRAS» Liste 3-15 N°2001-3905 (état 3) de la FEMA/FDA (liste des Substances Edulcorantes dites GRAS de la FEMA/FDA)} consistant en alcool benzylique.

2. Procédé selon la revendication 1, dans lequel la masse molaire de la polyvinyl-pyrrolidone est comprise entre 10 000 et 60 000, de préférence entre 30 000 et 50 000.

3. Procédé selon la revendication 1 ou 2, dans lequel la fraction de composant masquant les odeurs (A) forme de 0,001 à 50% en poids, de préférence 0,1 à 20% en poids, de la composition masquant les odeurs.

4. Procédé selon une ou plusieurs des revendication 1 à 3, dans lequel la composition masquant les odeurs contient en outre une matière odorante fonctionnelle (B), qui, de préférence, est constituée de un, ou de façon encore plus préférée de plusieurs des substances suivantes :

   butyratehexyl, acétylacétate, isobutylisobutyrate, acétate de cis-3-héxéne-1-yle, γ-décalactone, éthylcaproate, butylacétate, éthylbenzoate, éthylbutyrate, hexylacétate, méthylcaproate, alcool phényléthylique, citronellol, aldéhyde undécylique, benzylphénylacétate, alcool cinnamique, eugénol, acétate benzylique, linalool, cis-jasmone, anthranilate d'acétylméthyle, cis-3-hexéne-1-ol, salicylate cis-3-hexéne-1-ylique, méthylbenzoate, salicylate de méthyle, acétate de géranyle, acétate de cis-3-hexéne-1-ylique, litsea cubeba, alcool phénylpropylique et phényléthylacétate.

5. Procédé selon la revendication 4, dans lequel la matière odorante fonctionnelle (B) forme de 0,001 à 20% en poids, de préférence 0,1 à 5% en poids, de la composition masquant les odeurs.

6. Procédé selon une ou plusieurs des revendications 1 à 5, dans lequel le composant à base de matière aromatique (C) forme de 0,001 à 95% en poids, de préférence 0,1 à 80% en poids, de la composition masquant les odeurs.

7. Procédé selon une ou plusieurs des revendications 1 à 6, dans lequel le composant à base de matière aromatique (C) contient au moins un constituant aromatique GRAS et en particulier un alcool aromatique GRAS et/ou un constituant polyphénolique GRAS.

8. Procédé selon une ou plusieurs des revendications 1 à 7, dans lequel le composant à base de matière aromatique (C) contient

   a) un ou plusieurs alcools aromatiques GRAS ou leurs constituants
   b) un ou plusieurs constituants aromatiques choisis dans le groupe comprenant :

      (b1) des constituants polyphénoliques, et
      (b2) des acides aromatiques GRAS ou leurs constituants,

dans lequel le composant à base de matière aromatique (C), contient de préférence

    de 0,1 à 99 % en poids, de préférence de 0,5 à 99 % en poids du composant (a),
    de 0 à 25 % en poids, de préférence de 0,01 à 10 % en poids du composant (b1) et
    de 0 à 70 % en poids, de préférence de 0,01 à 30 % en poids du composant (b2).

9. Procédé selon la revendication 8, dans lequel :

- l'alcool aromatique GRAS (a) est choisi dans le groupe comprenant:

    l'alcool benzylique, l'acétoïne, l'alcool éthylique, l'alcool propylique, l'alcool isopropylique, le propylène glycol, la glycérine, l'alcool n-butylique, l'alcool iso-butylique, l'alcool hexylique, le L-menthol, l'alcool octylique, l'alcool cinnamique, l'alcool n-méthylbenzylique, l'alcool heptylique, l'alcool n-amylique, l'alcool iso-amylique, l'alcool anisique, le citronellol, l'alcool n-décylique, le géraniol, le β-γ-hexenol, l'alcool laurique, le linalol, le nérolidol, le nonadienol, l'alcool nonylique, le rhodinol, le terpinéol, le bornéol, le clinéol, l'anisole, l'alcool cuminique, le 10-undecen-1-ol et le 1-hexadécanol et leurs constituants;

- le constituant polyphénolique (b1) est choisi dans le groupe comprenant:

    la pyrocatéchine, la résorcine, l'hydroquinone, la phloroglucine, le pyrogallol, le cyclohéxane, l'acide usnique, les polyphénols acyliques, les lignines, l'anthocyane, les flavones, les catéchines, les constituants d'acide gallique, l'acide caféique, les flavonoïdes, les constituants des polyphénols mentionnés et les extraits de camellia primula et

- l'acide GRAS (b2) est choisi dans le groupe comprenant:

    l'acide acétique, l'acide aconitique, l'acide adipinique, l'acide formique, l'acide de pomme, l'acide caprique, l'acide nonylique, l'acide phenoxyacétique, l'acide hydrocinnamique, l'acide pélargonique, l'acide lactique, l'acide phénoxyacétique, l'acide phénylacétique, l'acide valérianique, l'acide iso-valérianique, l'acide cinnamique, l'acide citrique, l'acide d'amandes, l'acide tartrique, l'acide fumarique, l'acide tannique et leurs constituants.

10. Procédé selon une ou plusieurs des revendications 7 à 9, dans lequel la substance contient

    a1) un alcool aromatique GRAS , en particulier l'alcool benzylique, en tant que constituant essentiel et le cas échéant
    a2) un ou plusieurs alcools aromatiques GRAS supplémentaires ou leurs constituants
    (b1) un ou plusieurs constituants polyphénoliques, et/ou
    (b2) un ou plusieurs acides GRAS ou leurs constituants,

le composant à base de matière aromatique (C), contenant de préférence

    de 0,1 à 99 % en poids, de préférence de 0,1 à 75 % en poids d'alcool benzylique;
    de 0 à 99,8 % en poids, de préférence de 0,01 à 9 % en poids du composant (a2) et
    de 0 à 25 % en poids, de préférence de 0,01 à 10 % en poids du composant (b1) et
    de 0 à 70 % en poids, de préférence de 0,01 à 30 % en poids du composant (b2).

11. Procédé selon une ou plusieurs des revendications 1 à 7, dans lequel le composant à base de matière aromatique contient des constituants aromatiques GRAS supplémentaires, choisis dans le groupe comprenant

    (c) des phénols,
    (d) des esters,
    (e) des terpènes,
    (f) des acétals,
    (g) des aldéhydes et
    (h) des huiles éthérées,

le composant à base de matière aromatique contenant de préférence de 0,001 à 25 % en poids , de préférence de 0,01 à 9 % en poids de constituants aromatiques GRAS supplémentaires (c) à (h).

12. Procédé selon la revendication 11, dans lequel les constituants aromatiques GRAS supplémentaires sont des phénols (c) et/ou des huiles éthérées (h). (h).

13. Procédé selon une ou plusieurs des revendications 1 à 7, dans lequel le composant à base de matière aromatique ne contient pas de dérivés de constituants aromatiques GRAS.

14. Procédé selon une ou plusieurs des revendications 10 à 13, dans lequel le composant à base de matière aromatique contient un ou deux alcools aromatiques GRAS (a2) et au moins un constituant polyphénolique (b1), ledit constituant polyphénolique consistant en tanin.

**15.** Procédé selon la revendication 14, dans lequel le composant à base de matière aromatique contient de 0,1 à 20% en poids d'alcool benzylique et de 0,01 à 10% en poids de tanin.

**16.** Procédé selon une ou plusieurs des revendications 1 et 7 à 15, dans lequel la composition contient en outre un ou plusieurs alcools polyvalents comprenant 2 à 10 atomes de C, des émulsifiants, des stabilisateurs, des antioxydants, des agents de conservation, des solvants et/ou des substances porteuses.

**17.** Procédé selon une ou plusieurs des revendications 7 à 16, dans lequel la pulvérisation dans l'air de la composition masquant les odeurs est réalisée par un système à double injection de matières, un système de vaporisation, un appareil de formation de bulles ou un appareil à mèches, dans lequel:

（i) la proportion de composition masquant les odeurs du mélange d'air et de composition masquant les odeurs amenée dans la zone à traiter est de 10 à 100 ppm; ou
(ii) la dose de composition masquant les odeurs distribuée ou pulvérisée est de 0,000001 à 1ml par m$^3$ d'air par heure, de préférence de 0,001 à 0,1ml par m$^3$ d'air par heure

**18.** Composition masquant les odeurs pour la réduction d'odeurs non toxiques telle que décrite dans les revendication 1 à 16, dans laquelle ladite composition masquant les odeurs contient au moins :

- un composant masquant les odeurs (A) choisi dans le groupe comprenant amidon de maïs, sels de manganèse et polyvinyl-pyrrolidone, ladite composition masquant les odeurs contenant cependant au moins de la polyvinylpyrrolidone et
- un composant à base de matière aromatique (C) choisie dans le groupe comprenant les huiles éthérées, les constituants aromatiques et les parfums,

le composant à base de matière aromatique (C), contenant cependant au moins le constituant aromatique dit GRAS {Généralement Considéré Comme Sûr selon «Flavour Substances GRAS» Liste 3-15 N°2001-3905 (état 3) de la FEMA/FDA (liste des Substances Edulcorantes dites GRAS de la FEMA/FDA)} consistant en alcool benzylique, à la condition que cette composition, lorsqu'elle contient 0,5 à 5,0% en poids de polyvinyl-pyrrolidone ne contienne pas 0,1 à 10,0% d'un acide naphtalène sulfonique et/ou d'un de ses sels.

**19.** Application d'une composition masquant les odeurs telle que décrite dans la revendication 18.

FIG.1

FIG.2

Ventilator

Verdampfung

Produktausbringung

Erhitzte Aluschale

Dosierung
Zeitschaltuhr

Pumpe

Produkt

FIG.3

FIG.4

FIG.5

FIG. 6

**Ventilator**

Verdampfung

Produktausbringung

**Heizplatine**

**Docht**

**Produkt**

FIG.7